Europäisches Patentamt

European Patent Office

Office européen des brevets

(19)

(11) Publication number: **0 105 521**
**A2**

(12) # EUROPEAN PATENT APPLICATION

(21) Application number: **83109896.7**

(22) Date of filing: **04.10.83**

(51) Int. Cl.³: **C 12 N 15/00**
**C 12 P 21/02**

(30) Priority: **05.10.82 JP 175465/82**

(43) Date of publication of application:
**18.04.84 Bulletin 84/16**

(84) Designated Contracting States:
**AT BE CH DE FR GB IT LI NL SE**

(71) Applicant: **Takeda Chemical Industries, Ltd.**
**27, Doshomachi 2-chome Higashi-ku**
**Osaka-shi Osaka, 541(JP)**

(72) Inventor: **Igarashi, Koichi**
**C3-303, 3 Takenodai**
**Nagaokakyo Kyoto 617(JP)**

(72) Inventor: **Kurokawa, Tsutomu**
**1-50, Suimeidai 1-chome**
**Kawanishi Hyogo 666-01(JP)**

(72) Inventor: **Sasada, Reiko**
**3-811, 48-3, Mibubojo-cho**
**Nakagyo-ku Kyoto 604(JP)**

(74) Representative: **von Kreisler, Alek, Dipl.-Chem. et al,**
**Deichmannhaus am Hauptbahnhof**
**D-5000 Köln 1(DE)**

(54) Novel DNA, microorganism transformed therewith and use thereof.

(57) Peptide can be obtained, not in the form of a chimera peptide with rec A protein, by growing a transformant which contains a DNA containing one or more rec A promoters, at the 3'-end of the rec A promoter or promoters no translational start codon ATG being directly linked, and containing downstream from the rec A promoter or promoters a peptide-encoding nucleotide beginning with a translational start codon and recovering the peptide.

EP 0 105 521 A2

- 1 -

Novel DNA, Microorganism Transformed Therewith
and Use Thereof

This invention relates to a novel DNA. More particularly, this invention relates to a promoter for gene expression.

Advances in gene manipulation technique have led researchers to attempt the production of various substances on a large scale and at a low cost, which can hardly be in large amount from natural sources, by causing microorganisms to produce such substances as gene products. In this case, the amount of gene product by microorganisms largely depends on the activity of a promoter which lies upstream from a relevant structural gene. Thus, the promoter controls the gene transcription and therefore, for increasing the gene product productivity, it is very important to search for a potent promoter for use in the production of various gene products. Typical promoters so far used for gene expression are tryptophan (trp) promoter, $\lambda P_L$ promoter and lactose (lac) promoter, among others.

On the other hand, the Escherichia coli rec A promoter is a promoter which controls the synthesis of the rec A gene product. The rec A gene plays a main role in the so-called SOS response [Witkin, E. M., Bacteriol. Reviews, 40, 869 (1976)] which involves the induced synthesis of its product and the repair of damaged DNA upon damage of DNA in cells or upon inhibition of replication. Its product (rec A protein) has a proteolytic

- 2 -

activity. Since the lex A gene product acts as the rec A gene repressor, the expression of the rec A gene is normally suppressed. However, a DNA-damaging treatment (e.g. UV irradiation, nalidixic acid treatment, mitomycin treatment) strongly induces the synthesis of the rec A protein. This indicates that the rec A promoter is activated by such treatment. Therefore, if a peptide-encoding nucleotide is inserted downstream from the rec A promoter, the synthesis of its product can be controlled by the above treatment. Such is a very advantageous property for the production of a peptide. Another characteristic feature of the rec A promoter consists in that the promoter activity is very strong.

The base sequence of the rec A promoter has already been reported [Horii, T. et al., Proc. Natl. Acad. Sci. U.S.A., 77, 313 (1980); Sancar, A. et al., ibid., 77, 2611 (1980)]. However, there is no adequate restriction enzyme cleavage site downstream from the rec A promoter. Accordingly, it is difficult to insert, downstream from this promoter as it is, a peptide-encoding nucleotide. Since there is no restriction enzyme recognition site immediately following the rec A promoter, the ligation of a peptide-encoding nucleotide downstream therefrom results in the production of a chimera peptide of the rec A protein with the structural gene product. Therefore, no positive attempt to make use of the rec A promoter in gene expression has so far been reported. The present inventors prepared the rec A promoter and introduced various restriction enzyme recognition sites on both ends thereof, and succeeded in reconstructing a promoter suitable for direct expression of an intended peptide-encoding nucleotide inserted downstream therefrom, not in the form of a chimera peptide between the rec A protein and the polypeptide.

Thus, the present invention provides a DNA which contains one or more rec A promoters, at the 3'-end of the promoter or promoters no translational start codon ATG being directly linked, a transformant containing said DNA and a method of producing a peptide by cultivation of a transformant which contains a DNA containing one or more rec A promoters, at the 3'-end of the rec A promoter or promoters no translational start codon ATG being directly linked, and containing downstream from the rec A promoter or promoters a peptide-encoding nucleotide beginning with a translational start codon other than the rec A structural gene.

In the present specification, claims and drawings, the DNA having the nucleotide sequence as shown in Figure (hereinafter referred to as Fig.) 1·is referred to as "rec A promoter" for convenience' sake.

The symbols as used in the present specification, claims and drawings each has the meaning given in Table 1.

Table 1

| | |
|---|---|
| DNA | deoxyribonucleic acid |
| A | deoxyadenylate |
| T | deoxythymidylate |
| G | deoxyguanylate |
| C | deoxycytidylate |
| RNA | ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediamine tetraacetate |
| b | base(s) |
| bp | base pair(s) |
| kbp | kilobase pairs |
| kb | kilobases |
| Apc$^r$ | ampicillin resistance |
| Tet$^r$ | tetracycline resistance |
| recAp | rec A promoter |

The rec A promoter reconstruction and the expression plasmid construction are performed, for example in the following manner.

Double digestion of the plasmid pMCR611 DNA [Miki, T. et al., Mol. Gen. Genet., 183, 25 (1981)] with the restriction enzymes BamHI and PstI gives a 1.25 Kb DNA fragment containing the rec A promoter. Digestion of said fragment with the restriction enzyme HaeIII gives a 300 b DNA fragment containing the rec A promoter. Further digestion of this 300 b DNA fragment with the restriction enzyme HpaII gives a 148 b DNA fragment covering, according to the report of Sancar et al. (vide supra), the sequence from -129 to 19 with the RNA transcription startpoint on the rec A promoter being counted as 1. The SD sequence of the rec A promoter is missing in said DNA fragment.

Separately, oligonucleotides (dCGGTATTACCCGGC, dATGACAGGAGTAAAAG, dTCATGCCGGGTAATAC, dGATCCTTTTACTCCTG) are chemically synthesized for example by the phosphotriester method [Crea, R. et al., Proc. Natl. Acad. Sci. U.S.A., 75, 5765 (1978)]. These are ligated together, for example with T4 DNA ligase. The thus-obtained 30 b DNA fragment is joined to the above 148 b DNA, for example with T4 DNA ligase, to give a 178 b DNA fragment. The cohesive termini on both ends of this DNA are filled in, for example with DNA polymerase I large fragment and then the BamHI linker (dCCGGATCCGG) is joined to said DNA using T4 DNA ligase. The resulting DNA fragment is digested with the restriction enzyme BamHI and then inserted into the plasmid pBR322 digested with BamHI with the use of T4 DNA ligase. In this way, there can be constructed a recombinant plasmid, pREC3, which contains the rec A promoter possessing BamHI recognition sites on the ends thereof. An expression plasmid, pREC33, which possesses a BamHI site only downstream from the rec A promoter, can also be constructed by eliminating another BamHI site, which lies upstream from the rec A promoter, from said pREC3, for example by a series of operational steps, for example, partial digestion of pREC3 with BamHI, repair of the cohesive termini with DNA polymerase I large fragment and rejoining with T4 DNA ligase.

It is also possible to cut out the rec A promoter of pREC3 with the restriction enzyme BamHI, render the cohesive termini on both ends blunt with S1 nuclease, DNA polymerase I large fragment or the like and join thereto the EcoRI, ClaI or HindIII linker, for instance, whereby a DNA possessing a restriction enzyme recognition site suitable for the insertion of a variety of peptide-encoding nucleotides into said site can be introduced into the promoter at the end thereof. In this case, the number of bases as a spacer (inclusive of linker) between the rec A promoter and a translational start codon is 1 to 20, more preferably 3 to 15 and most preferably 6 to 13.

Furthermore, it is possible to cut out, with a restriction enzyme, the rec A promoter of pREC3 or corresponding plasmids with the various recognition sites as described above, to ligate molecules of the cut-out promoter with each other with T4 DNA ligase and to insert the thus-obtained successive dimer, trimer, etc. into the plasmid pBR322, whereby an expression plasmid having still more potent promoter activity can be obtained.

Further, the expression plasmid may contains, in addition, such a promoter as bla, tufB and lpp promoter.

Any nucleotide coding for a peptide (e.g. human immunoglobulin E H-chain polypeptide, hepatitis B virus surface antigen, human growth hormone, somatostatin) can be inserted, with the aid of a restriction enzyme and T4 DNA ligase, into the expression vector constructed in the above manner.

The above expression plasmid or a recombinant DNA obtained by insertion of an optionally selected peptide-encoding nucleotide thereinto can be introduced into an adequate host organism. The host organism includes

- 6 -

microorganisms such as _Escherichia coli_, _Bacillus subtilis_ and yeasts. Preferred are strains of _Escherichia coli_ (strains 294, W3110, RR1, DM511, DM1187, etc.), in particular strain 294. The DM511 strain containing mutation in the lex A gene which is the repressor against the rec A promoter [Mount, D. W. et al., J. Bacteriol., 116, 950 (1973)] and the DM1187 strain [Mount, D. W. et al., Proc. Natl. Acad. Sci. U.S.A., 74, 300 (1977)] are also suitable for the development of activity by the present promoter.

The transformation of a host organism with the above recombinant DNA can be carried out by the known method [Cohen, S. N. et al., Proc. Natl. Acad. Sci. U.S.A., 69, 2110 (1972)] or a modification thereof. Thus, for instance, _Escherichia coli_ strain 294 can be transformed by introduction thereinto of the recombinant plasmid DNA obtained in the above manner. An adequate transformant can be selected with the ampicillin or tetracycline resistance as the indicator. The strain 294 is a known strain [Backman, K. et al., Proc. Natl. Acad. Sci. U.S.A., 73, 4174 (1976)]. It has also been deposited with the Institute for Fermentation, Osaka under No. IFO-14171.

The thus-selected transformant is cultivated in a _per se_ known medium. As the medium, there may be mentioned L broth and glucose- and casamino acids-containing M-9 medium, among others. For efficient functioning of the promoter, such an agent as nalidixic acid and mitomycin C may be added as necessary.

The cultivation is conducted generally at 15° to 43°C, preferably 28° to 42°C, for 2 to 24 hours, preferably 4 to 16 hours. Aeration and/or stirring may be made as necessary.

After cultivation of the above transformant, cells are harvested by the known method, then suspended for example in a buffer solution, and destructed for example by treatment with lysozyme, a surface active agent or ultrasonic waves. The supernatant is collected by centrifugation. The isolation of the peptide from said supernatant may be effected by following the generally known protein purification method.

In this manner, the desired peptide itself can be obtained directly, not in the form of a chimera peptide between the rec A protein and the peptide.

Furthermore, it is possible to insert the rec A promoter into an optionally selected plasmid upstream from, for example, the ampicillin resistance gene (gene coding for $\beta$-lactamase) in the same orientation as said gene, whereby the promoter activity can be measured with the increased $\beta$-lactamase activity as the indicator. The $\beta$-lactamase activity can be determined, for example by using cephaloridine as the substrate, adding a cell suspension thereto and determining the decomposition of cephaloridine in terms of a decrease in absorbance (at 260 nm) [Ross and O'Callaghan, Methods in Enz., 43, 69 (1975)].

Brief description of the Drawings

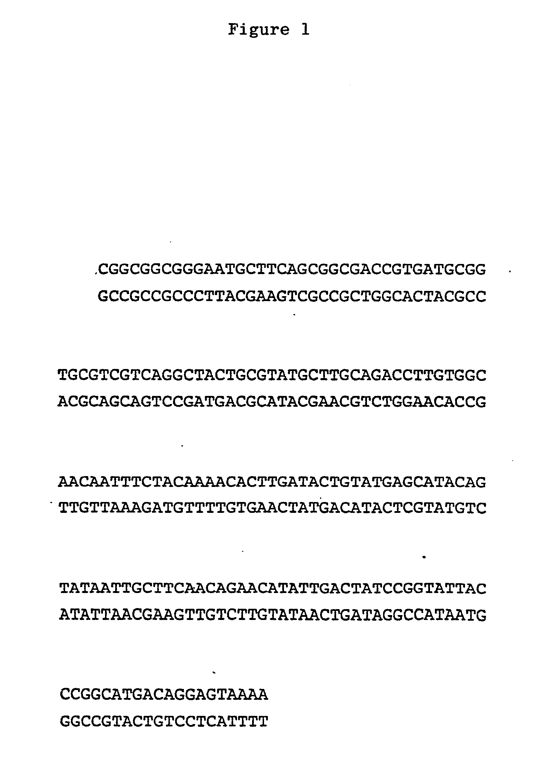

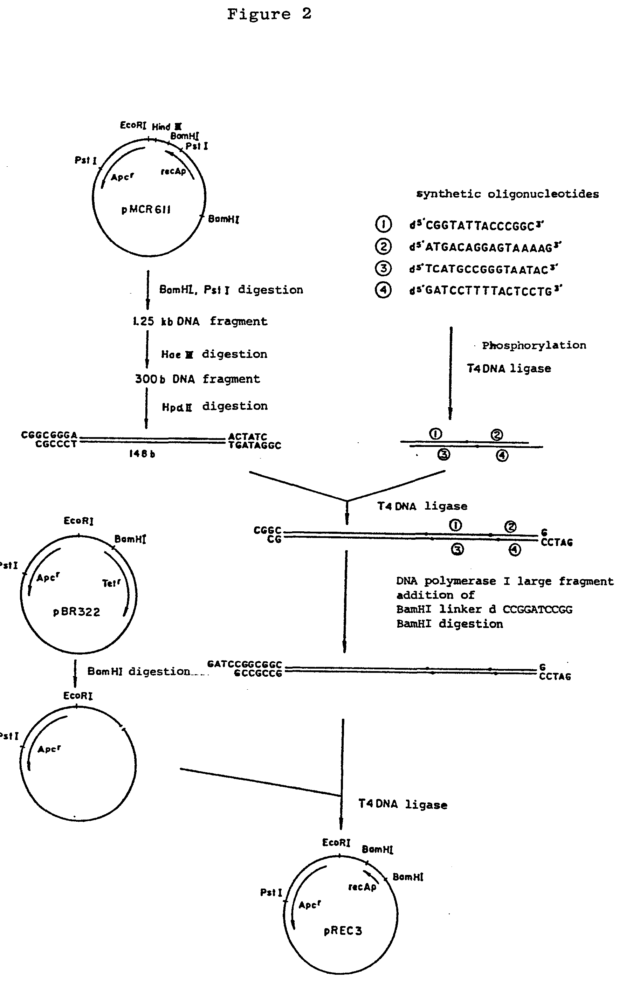

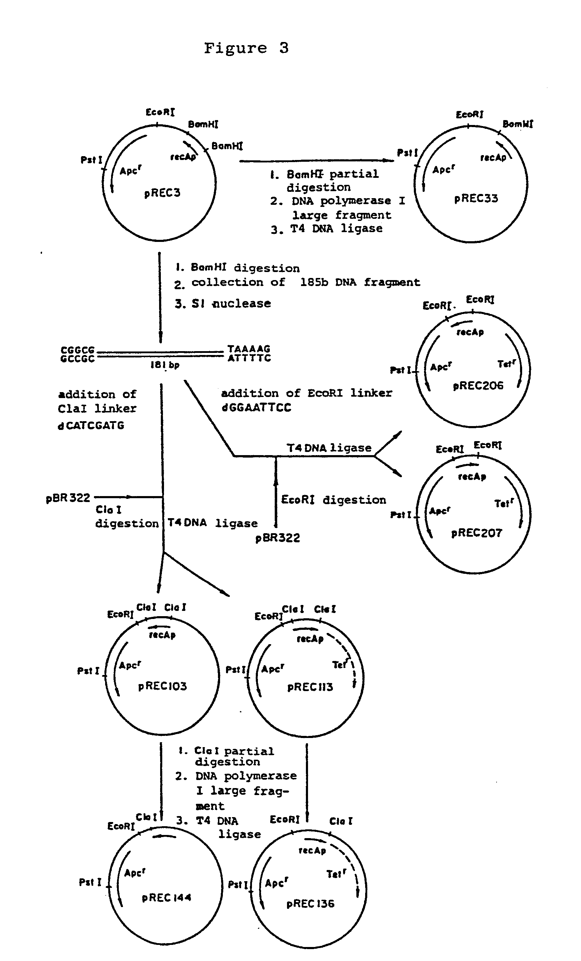

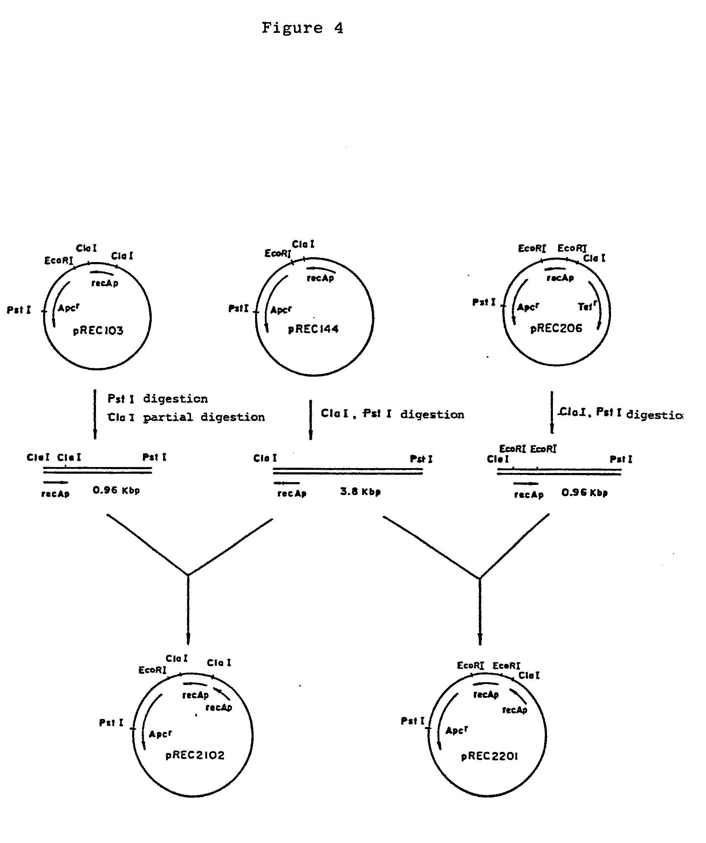

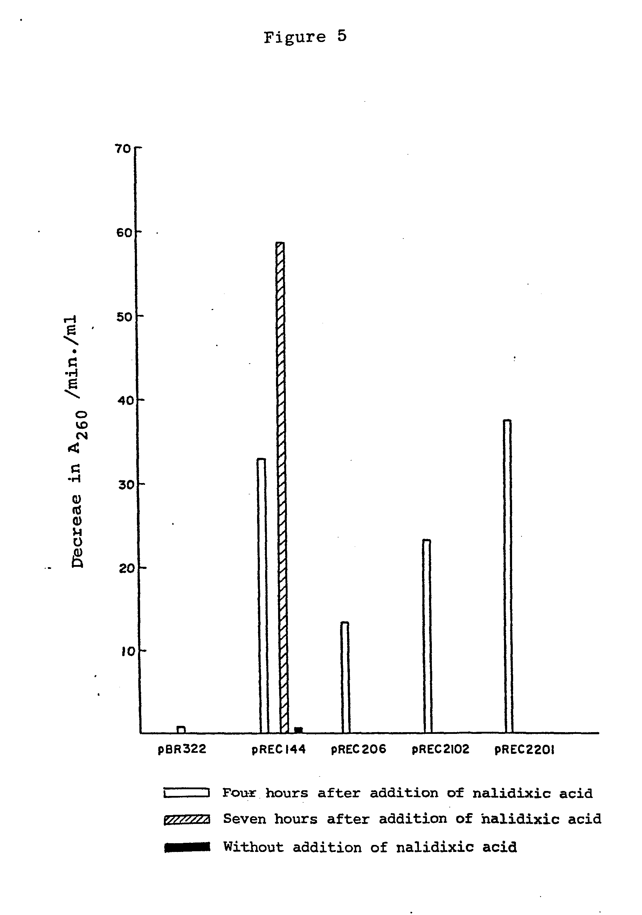

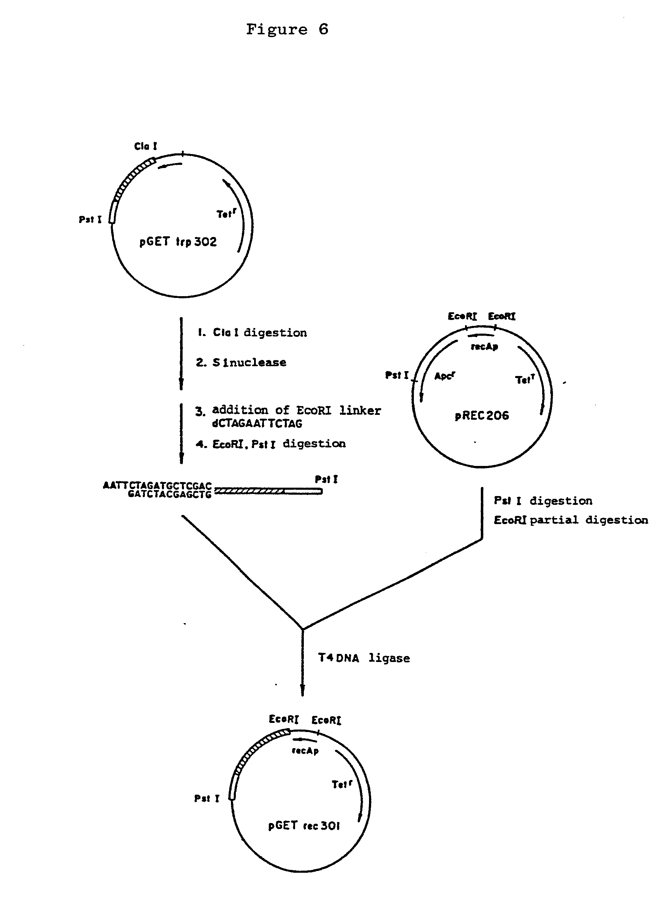

Fig. 1 illustrates the rec A promoter DNA. Fig. 2 shows the construction scheme for pREC3 and Fig. 3 shows the construction scheme for pREC33, pREC206, pREC207, pREC103, pREC113, pREC114 and pREC136. Fig. 4 shows the construction scheme for expression vectors pREC2102 and pREC2201 each containing two successive rec A promoters. Fig. 5 shows the promoter activity data as measured and Fig. 6 shows the construction scheme for a human immunoglobulin E (IgE) H-chain gene expression plasmid. The portion indicated by ▨▨▨▨ represents the human IgE H-chain polypeptide-encoding DNA fragment.

The following examples are given for further illustration of the present invention. It is to be noted that they are by no means limitative of the present invention.

## Example 1

Preparation of rec A promoter-containing DNA fragment
(cf. Fig. 2)

The plasmid pMCR611 (300 µg) (vide supra) was
digested with the restriction enzymes BamHI and PstI
(Takara Shuzo). Agarose gel (1%) electrophoresis gave a
1.25 kbp DNA fragment containing the rec A promoter portion,
which fragment was eluted from the gel [McDonell, M. W.
et al., J. Mol. Biol., 110, 119 (1977)]. This DNA
fragment was purified by phenol extraction, ether
extraction and ethanol precipitation, then digested with
the restriction enzyme HaeIII (Takara Shuzo), and fractionated by 1.5% agarose gel electrophoresis to give a
300 b DNA fragment containing the rec A promoter portion.
The fragment was purified in the same manner as mentioned
above. This DNA fragment was further digested with the
restriction enzyme HpaII (Takara Shuzo). The digestion
product was subjected to 8% acrylamide gel (acrylamide:
bisacrylamide = 19:1) electrophoresis so as to isolate the
148 b DNA fragment covering the sequence from -129 to 19
with the RNA transcription startpoint on the rec A
promoter being counted as 1 as reported by Sancar et al.
(vide supra). The thus-fractionated fragment was purified
in the same manner as above.

Separately, four oligonucleotides each containing a
portion of the 3'-end of the rec A promoter, namely (1)
dCGGTATTACCCGGC, (2) dATGACAGGAGTAAAAG, (3) dTCATGCCGGGTAATAC
and (4) dGATCCTTTTACTCCTG, were chemically synthesized by
the phosphotriester method (vide supra). Of these oligonucleotides, (2) and (3) were phosphorylated at the 5'-
end using polynucleotide kinase [New England Biolabs (NEB)]
and ATP. Equal amounts (0.5 µg) of the four oligonucleo-
tides, namely oligonucleotides (1) and (4) and 5'-
phosphorylated oligonucleotides (2) and (3), were mixed
and, using T4 DNA ligase, they were ligated to one another
by maintaining the mixture in 50 µl of reaction medium

(66 mM Tris-HCl, pH 7.6, 6.6 mM $MgCl_2$, 10 mM dithiothreitol, 1 mM ATP, 300 units T4 DNA ligase) at 14°C for 16 hours. The reaction product was fractionated by 10% acrylamide gel (acrylamide : bisacrylamide = 19:1) electrophoresis. The 30 b DNA fragment, a ligation product of the oligonucleotides, was isolated and purified in the same manner as mentioned above.

In the next place, the above-mentioned 148 b DNA fragment and the 30 b DNA fragment were ligated together using T4 DNA ligase. The reaction product was fractionated by 8% acrylamide gel electrophoresis. The 178 b DNA fragment which thus formed was isolated and purified. The cohesive termini of this DNA fragment were rendered blunt by contacting the DNA fragment with DNA polymerase I large fragment [Bethesda Research Laboratories (BRL)] in 30 μl of reaction mixture (40 mM potassium phosphate buffer, pH 7.5, 6.6 mM $MgCl_2$, 1 mM 2-mercaptoethanol, 20 μM dATP, 20 μM dGTP, 20 μM dCTP, 20 μM dTTP, 2 units DNA polymerase I large fragment) at 20°C for 30 minutes. The product DNA was purified by phenol extraction and ethanol precipitation and thereto was joined the BamHI linker dCCGGATCCGG (BRL) phosphorylated at the 5'-end, using T4 DNA ligase. The product was digested with the restriction enzyme BamHI and inserted into the pBR322 DNA BamHI digestion product. The resultant recombinant DNA was used for transformation of Escherichia coli strain 294. Ampicillin-resistant, tetracycline-sensitive transformants were selected and plasmid DNAs were isolated therefrom according to the method of Birnboim-Doly [Nucleic Acids Res., 7, 1513 (1973)]. A plasmid which gave a 185 b DNA fragment upon digestion with the restriction enzyme BamHI was selected. At the same time, the base sequence of the 185 b DNA fragment thus cut out was determined according to the dideoxynucleotide synthetic chain termination method [Nucleic Acids Res., 9, 309 (1981)]. In this manner, there was obtained the product of ligation of the 148 b DNA fragment from the 5'-end portion of the rec A promoter with the BamHI linker as joined together in the expected manner. Its nucleotide sequence was as follows:

```
      -130      -120      -110      -100
       |         |         |         |
GATCCGGCGGCGGGAATGCTTCAGCGGCGACCGTGATGCGG
      GCCGCCGCCCTTACGAAGTCGCCGCTGGCACTACGCC


      -90       -80       -70       -60
       |         |         |         |
TGCGTCGTCAGGCTACTGCGTATGCTTGCAGACCTTGTGGC
ACGCAGCAGTCCGATGACGCATACGAACGTCTGGAACACCG


      -50       -40       -30       -20
       |         |         |         |
AACAATTTCTACAAAACACTTGATACTGTATGAGCATACAG
TTGTTAAAGATGTTTTGTGAACTATGACATACTCGTATGTC


      -10        1        10        20
       |         |         |         |
TATAATTGCTTCAACAGAACATATTGACTATCCGGTATTAC
ATATTAACGAAGTTGTCTTGTATAACTGATAGGCCATAATG


30            40
 |             |
CCGGCATGACAGGAGTAAAAG
GGCCGTACTGTCCTCATTTTCCTAG
```

As compared with the report of Sancar, A et al., the above sequence contains one more base pair ($_T^A$) at -121.

A pBR322 plasmid with this DNA fragment inserted therein with the same orientation of the rec A promoter with that of the ampicillin resistance-offering β-lactamase gene was named pREC3.

Example 2

Construction of rec A promoter-containing expression vector (cf. Fig. 3)

(i) Expression vector with restriction enzyme BamHI recognition site as a site for insertion.

The expression vector plasmid pREC3 containing the site for insertion at the restriction enzyme BamHI recognition site was partially digested with the restriction enzyme BamHI. The resultant cohesive termini of the plasmid DNA cleaved at one site of the plasmid were rendered blunt according to the method described in Example 1 using DNA polymerase I large fragment and rejoined together using T4 DNA ligase. The resultant DNA was used for transformation of Escherichia coli strain 294. Ampicillin-resistant transformants were selected, and plasmid DNAs were isolated therefrom according to the method of Birnboim-Doly (alkali extraction method). A plasmid in which the BamHI site on the 5'-end (upstream) of the rec A promoter was eliminated from the restriction enzyme cleavage pattern was selected and named pREC33.

(ii) Expression vector with restriction enzyme ClaI recognition site as a site for insertion.

The plasmid pREC3 was digested with the restriction enzyme BamHI, followed by 6% acrylamide gel electrophoresis, which gave a 185 b rec A promoter unit DNA. The cohesive termini of this DNA were rendered blunt with S1 nuclease (BRL) in 100 μl of reaction mixture (0.5 μg DNA, 0.03 M sodium acetate buffer, pH 4.5, 0.3 M NaCl, 4.5 mM $ZnCl_2$, 3 units S1 nuclease) at 22°C for 30 minutes and then the DNA was purified by phenol extraction and ethanol precipitation. The ClaI linker dCATCGATG (chemically synthesized by the phosphotriester method) was phosphorylated at the 5'-end and ligated with the above DNA using T4 DNA ligase. The ligation product was digested with the restriction enzyme ClaI (Boehringer) and ligated with the pBR322 DNA digested with the same enzyme. The ligation product was used for transformation of Escherichia coli strain 294. Ampicillin-resistant transformants were selected. Plasmid DNAs were isolated from these transformants according to the method of Birnboim-Doly (vide supra). Based on the restriction enzyme cleavage pattern,

- 12 -

a plasmid (pREC103) containing the rec A promoter in the same orientation as that of the β-lactamase gene and a plasmid (pREC113) containing the rec A promoter in the opposite orientation to the β-lactamase gene were selected. In pREC113, the rec A promoter is directed toward the tetracycline resistance gene, so that the transformant carrying this plasmid is partially resistant to tetracycline.

pREC103 and pREC113 were partially cleaved with the restriction enzyme ClaI and, following the procedure of Example 2-(i), plasmids with the ClaI recognition site on the 5'-end side of (upstream from) the rec A promoter eliminated were selected and named pREC144 and pREC136, respectively.

(iii) Expression vector with restriction enzyme EcoRI recognition site as a site for insertion

The plasmid pREC3 was digested with the restriction enzyme BamHI and, following the procedure of Example 2-(ii), a rec A promoter unit DNA was isolated and rendered blunt-ended. This DNA was ligated with the EcoRI linker dGGAATTCC (BRL) phosphorylated at the 5'-end, using T4 DNA ligase, followed by digestion with the restriction enzyme EcoRI (Takara Shuzo) and ligation with the pBR322 DNA digested with the same enzyme using T4 DNA ligase. The ligation product was used for transformation of Escherichia coli strain 294, and ampicillin- and tetracycline-resistant transformants were selected. Plasmid DNAs were isolated from these transformants according to the method of Birnboim-Doly (vide supra) and, based on the restriction enzyme cleavage pattern, a plasmid (pREC206) with the rec A promoter in the same orientation as the β-lactamase gene and one (pREC207) with the rec A promoter in the opposite orientation to said gene were selected.

### Example 3

Construction of expression vector with successive rec A promoters (cf. Fig. 4)

The plasmid pREC144 constructed in Example 2 was double-digested with the restriction enzymes ClaI and PstI, followed by 1.2% agarose gel electrophoresis. Then, a 3.8 kbp DNA fragment containing the rec A promoter portion was isolated and purified according to the same method as described in Example 1.

The plasmid pREC103 constructed in Example 2 was separately digested with the restriction enzyme PstI and further digested partially with the restriction enzyme ClaI, and a 0.96 kbp DNA fragment containing the rec A promoter portion was isolated according to the above-mentioned method. This fragment was ligated with the above-mentioned 3.8 kbp DNA fragment with T4 DNA ligase to give an expression plasmid, pREC2102, with two successive rec A promoters. With this plasmid, a gene desired to be expressed can be inserted thereinto at the ClaI recognition site.

Separately, the plasmid pREC206 constructed in Example 2 was double-digested with the restriction enzymes ClaI and PstI, and a 0.96 kbp DNA fragment containing the rec A promoter portion was isolated according to the above-mentioned method. This was ligated with the above-mentioned 3.8 kbp DNA fragment using T4 DNA ligase to give an expression plasmid, pREC2201, containing two successive rec A promoters. With this plasmid, a gene desired to be expressed can be inserted thereinto at the EcoRI recognition site.

### Example 4

Assay of expression vectors for promoter activity

Of the expression vectors constructed in Examples 2 and 3, those in which the orientation of the rec A promoter agrees with that of the β-lactamase gene (i.e. pREC3, pREC33, pREC103, pREC144, pREC206, pREC2102 and pREC2201) can be assayed for the promoter activity using the β-lactamase activity as the indicator. The β-lactamase activity can be determined by using cephaloridine as the substrate and measuring the decrease in absorbance at 260 nm which reflects the decomposition of cephaloridine.

Thus, transformants of Escherichia coli strain 294 with the above expression plasmids were incubated in M-9 medium containing 1% glucose and 0.4% Casamino acids for 3 hours, then nalidixic acid was added to the concentration of 50 µg/ml, and incubation was continued. Four hours after nalidixic acid addition, the culture broth was sampled and assayed for β-lactamase by the above-mentioned method. For pREC144, the activity seven hours after nalidixic acid addition was also given by way of example.

Cephaloridine was dissolved in 10 mM Tris-HCl, pH 7.6, plus 1 mM EDTA in the concentration of 25 µg/ml. To 3 ml of the solution, there was added 5-25 µl of the culture broth. After 15 seconds, 30 seconds and 1 minute of incubation at room temperature, decreases in absorbance at 260 nm were determined. The values obtained were converted to the decrease in absorbance at 260 nm in each minute per milliliter of medium, which were then averaged. The thus-obtained mean values are shown graphically in Fig. 5.

As in seen Fig. 5, the addition of nalidixic acid strongly induced the rec A promoter activity of each expression plasmid as evidenced by the potentiation of β-lactamase activity (for pREC144, about 70 times, seven hours after nalidixic acid addition).

Example 5

Construction of human immunoglobulin E H-chain gene expression plasmid (cf. Fig. 6)

The plasmid pGETtrp302 with the human immunoglobulin E H-chain gene inserted therein downstream from the tryptophan promoter described in Example 3 of European Patent Application No. 83 108 699.6 filed on September 3, 1983, was used. Escherichia coli 294/pGETtrp302 carrying the plasmid pGETtrp302 has been deposited with the Institute for Fermentation, Osaka under deposit No. IFO-14285. The

- 15 -

plasmid was cleaved with the restriction enzyme ClaI. The resultant cohesive termini were rendered blunt by contacting with S1 nuclease(BRL) in 100 µl of reaction mixture (2 µg DNA, 0.03 M sodium acetate buffer, pH 4.5, 0.3 M NaCl, 4.5 mM $ZnCl_2$, 12 units S1 nuclease) at 22°C for 30 minutes. This DNA was purified by phenol extraction and ethanol precipitation and then ligated with the EcoRI linker dCTAGAATTCTAG (chemically synthesized by the phosphotriester method and phosphorylated at the 5'-end) using T4 DNA ligase. The ligation product was double-digested with the restriction enzymes EcoRI and PstI, and a 1.2 kb DNA fragment was isolated by the subsequent 1% agarose gel electrophoresis and purified according to the method described in Example 1.

Separately, the plasmid pREC206 was digested with the restriction enzyme PstI and then further digested partially with the restriction enzyme EcoRI, the digestion product was subjected to 1% agarose gel electrophoresis, and a 3.8 kb DNA fragment was isolated and purified by the method described in Example 1. This DNA was ligated with the aforementioned 1.2 kb human immunoglobulin E H-chain gene using T4 DNA ligase. The ligation product was used for transformation of Escherichia coli strain 294, and tetracycline-resistant transformants were selected. Plasmid DNAs were isolated from these transformants according to the method of Birnboim-Doly (vide supra) and, based on the restriction enzyme cleavage pattern, an expression plasmid, pGETrec301, with the rec A promoter and the human immunoglobulin E H-chain gene interconnectedly inserted therein was obtained. Escherichia coli 294/ pGETrec301 has been deposited with the Institute for Fermentation, Osaka under deposit No. IFO-14287.

### Example 6

Expression of human immunoglobulin E H-chain gene in Escherichia coli and isolation of human immunoglobulin E H-chain polypeptide

The transformant carrying the human immunoglobulin E H-chain gene expression plasmid pGETrec301 as obtained in Example 5 was cultured in M-9 medium containing 1% glucose and 0.4% Casamino acids for 4 hours. Then, nalidixic acid was added in the concentration of 50 µg/ml, and incubation was continued for further 4 hours. Thereafter, cells were harvested and suspended in 1/50 volume of a solution containing 50 mM Tris-HCl, pH 8.0, 0.2 M NaCl, 0.01 M EDTA, 10% sucrose and 150 µg/ml lysozyme. The suspension was allowed to stand at 0°C for 50 minutes and then treated at 37°C for 2 minutes. Thereafter, the suspension was cooled back to 0°C and, after addition of 0.1% of Triton X-100, subjected to sonication, followed by centrifugation at 15,000 rpm (Servall, SS34 rotor) for 30 minutes. The supernatant thus obtained had an immunoglobulin E activity of 1.1 µg/ml as measured by the RIST (Radio immuno sorbent test) method [Immunology, 14, 265 (1968)]. The immunoglobulin E H-chain polypeptide can be isolated from this supernatant and purified as described in Example 5 of European Patent Application No. 83 108 699.6 filed on September 3, 1983, that is by using an anti-human immunoglobulin E monoclonal antibody column.

What is claimed is:

1.    A DNA which contains one or more rec A promoters, at the 3'-end of the rec A promoter or promoters no translational start codon ATG being directly linked.

2.    A DNA according to Claim 1, wherein a nucleotide or nucleotides possessing a restriction enzyme recognition site are linked at one or each end of the rec A promoter or promoters.

3.    A DNA according to Claim 1, wherein the number of the rec A promoters contained therein is one or two.

4.    A DNA according to Claim 1, wherein the rec A promoter is the nucleotide of the nucleotide sequence as shown in Figure 1.

5.    A DNA according to Claim 1, which contains down-stream from the rec A promoter or promoters a peptide-encoding nucleotide beginning with a translational start codon other than the rec A structural gene.

6.    A DNA according to Claim 5, wherein the translational start codon is ATG.

7.    A DNA according to Claim 5, wherein the peptide-encoding nucleotide codes for a peptide containing human immunoglobulin E H-chain polypeptide or a fragment thereof.

8.    A transformant which contains a DNA containing one or more rec A promoters, at the 3'-end of the rec A promoter or promoters no translational start codon ATG being directly linked.

9.    A transformant according to Claim 8, wherein the DNA contains downstream from the rec A promoter or promoters
a peptide-encoding nucleotide beginning with a translational
start codon other than the rec A structural gene.

10. A transformant according to Claim 8, which is
Escherichia coli.

11. The transformant according to Claim 8, which is
Escherichia coli 294/pGETrec301 (IFO-14287).

12. A method of producing a peptide, which comprises
 a) growing a transformant which contains a DNA containing one or more rec A promoters, at the 3'-end of the
rec A promoter or promoters no translational start codon
ATG being directly linked, and containing downstream from
the rec A promoter or promoters a peptide encoding
nucleotide beginning with a translational start codon
other than the rec A structural gene,
 b) accumulating said peptide in the culture, and
 c) isolating the same from said culture.

13. A method according to Claim 12, wherein the peptide
is a peptide containing human immunoglobulin E H-chain
polypeptide or a fragment thereof.

14. A method of producing a DNA containing one or more
rec A promoters, at the 3'-end of the rec A promoter or
promoters no translational start codon ATG being directly
linked, which comprises
 a) digesting a DNA containing the rec A promoter and
the rec A structural gene or a fragment thereof with a
restriction enzyme to give a nucleotide containing a
fragment of the rec A promoter of which the SD sequence
is missing, and
 b) ligating the thus obtained nucleotide with chemically
synthesized nucleotide or nucleotides containing the SD sequence to give a DNA containing the rec A promoter, at the 3'-end of the rec A promoter or promoters no translational start codon ATG being directly linked, and if desired,

  c)  ligating the DNA with a nucleotide or nucleotides possessing a restriction enzyme recognition site, and if desired,

  d)  inserting the DNA into a plasmid to give a recombinant plasmid containing one or  more rec A promoters, at the 3'-end of the rec A promoter or promoters, and if desired,

  e)  transforming a host organism with said recombinant plasmid, and if desired,

  f)  inserting a peptide-encoding nucleotide having a translational start codon at its 5'-end downstream from the rec A promoter or promoters in said recombinant plasmid.

15.  A method of preparing a transformant which contains a DNA containing one or more rec A promoters, at the 3'-end of the rec A promoter or promoters no translational start codon ATG being directly linked, which comprises transforming a host organism with said DNA.

Figure 1


.CGGCGGCGGGAATGCTTCAGCGGCGACCGTGATGCGG
GCCGCCGCCCTTACGAAGTCGCCGCTGGCACTACGCC


TGCGTCGTCAGGCTACTGCGTATGCTTGCAGACCTTGTGGC
ACGCAGCAGTCCGATGACGCATACGAACGTCTGGAACACCG


AACAATTTCTACAAAACACTTGATACTGTATGAGCATACAG
TTGTTAAAGATGTTTTGTGAACTATGACATACTCGTATGTC


TATAATTGCTTCAACAGAACATATTGACTATCCGGTATTAC
ATATTAACGAAGTTGTCTTGTATAACTGATAGGCCATAATG


CCGGCATGACAGGAGTAAAA
GGCCGTACTGTCCTCATTTT

Figure 2

Figure 3

Figure 4

Figure 5

Four hours after addition of nalidixic acid

Seven hours after addition of nalidixic acid

Without addition of nalidixic acid

Figure 6